# EUROPÄISCHE PATENTANMELDUNG

(11) **EP 1 298 197 A1**
(43) Veröffentlichungstag der Anmeldung: **02.04.2003**
(21) Anmeldenummer: 02450217.1
(22) Anmeldetag: 27.09.2002
(51) Int. Cl.: C12G 1/028, C12M 1/36

(54) **Verfahren und Vorrichtung zum Behandeln von Maische**

(30) Priorität: 28.09.2001 AT 745012001
(71) Anmelder: Stingel ITT OEG, 8461 Ehrenhausen (AT)
(72) Erfinder: Nowrouzi, Markus, 8641 Ehrenhausen (AT)
(74) Vertreter: Miksovsky, Alexander, Dipl.-Ing.

(57) **Zusammenfassung**

Bei einem Verfahren und einer Vorrichtung zum Behandeln von Maische, wobei von der in einen Behälter (1) gefüllten Maische (2) wenigstens ein physikalischer Parameter erfaßt wird und ein Gärverlauf der Maische in Abhängigkeit von dem Parameter geregelt wird, ist vorgesehen, daß die Gasmenge, insbesondere die CO₂-Menge und/oder der CO₂-Gehalt, des sich im Behälter (1) über der Maische (2) befindlichen bzw. bildenden Gases während der Gärung gemessen wird und einer Steuer- bzw. Regeleinrichtung (7) zur Steuerung bzw. Regelung der Temperatur der Maische (2) im Behälter (1) zugeführt wird, wodurch eine genauere Überwachung sowie Steuerung bzw. Regelung der Gärung einer Maische (2) möglich wird.

## Beschreibung

Die vorliegende Erfindung bezieht sich auf ein Verfahren zum Behandeln von Maische, wobei von der in einen Behälter gefüllten Maische wenigstens ein physikalischer Parameter erfaßt wird und ein Gärverlauf der Maische in Abhängigkeit von dem Parameter geregelt wird, wobei die Gasmenge, insbesondere die CO₂-Menge und/oder der CO₂-Gehalt, des sich im Behälter über der Maische befindlichen bzw. bildenden Gases während der Gärung gemessen wird, sowie auf eine Vorrich tung zum Behandeln von Maische in einem Behälter mit wenigstens einem Sensor zur Erfassung eines physikalischen Parameters der Maische und einer Steuer- bzw. Regeleinrichtung zur Beeinflussung des Gärverlaufes.

Ein Verfahren sowie eine Vorrichtung zum Behandeln von Maische ist beispielsweise aus der DE-A 41 29 174 bekannt, wobei zur Steuerung des Gärverlaufs bzw. Gärvorgangs der Maische der Druck im Behälter erfaßt und geregelt wird und die Maische nach dem Druckentspannungsverfahren behandelt wird. Ein Verfahren der eingangs genannten Art ist beispielsweise der EP-B 0 271 380 zu entnehmen, wobei auf die Feststellung und Steuerung von Parametern der alkoholischen Gärung abgezielt wird. Darüber hinaus ist es beispielsweise bekannt, die Temperatur einer Maische während des Gärvorgangs zu messen bzw. zu bestimmen und beispielsweise über einen Wärmetauscher konstant zu halten bzw. auf ein gewünschtes Niveau zu bringen. Nachteilig bei diesen bekannten Verfahren ist, daß sowohl die Bestimmung des Drucks als auch der Temperatur zu ungenau ist, um die tatsächliche Gärintensität festzustellen, da die Gärung von vielen weiteren Faktoren abhängig ist.

Die vorliegende Erfindung zielt nun darauf ab, ein Verfahren sowie eine Vorrichtung der eingangs genannten Art zur Verfügung zu stellen, mit welchem(r) ein genauere Überwachung sowie Steuerung bzw. Regelung der Gärung einer Maische bzw. allgemein eines vergärbaren Materials durch eine exaktere Feststellung der tatsächlichen Gärintensität erzielbar ist.

Zur Lösung dieser Aufgaben ist ein Verfahren der eingangs genannten Art im wesentlichen dadurch gekennzeichnet, daß in Abhängigkeit von der Gasmenge die Temperatur der Maische im Behälter geregelt bzw. gesteuert wird. Da erfindungsgemäß die Gasmenge, insbesondere die CO₂-Menge und/oder der CO₂-Gehalt, des sich im Behälter über der Maische befindlichen bzw. bildenden Gases während der Gärung gemessen bzw. allgemein ermittelt wird, gelingt eine exaktere Feststellung der tatsächlichen Gärintensität bzw. des tatsächlichen Gärverlaufs, wobei insbesondere die CO₂-Menge und/oder der CO₂-Gehalt nicht nur ein Maß für den Fortschritt der Gärung ist, sondern durch eine durch die Bestimmung der CO₂-Menge oder des CO₂-Gehalts mögliche Steuerung bzw. Regelung des Gärverlaufs auch die Vermehrung bzw. Entwicklung der für die Gärung verantwortlichen Mikroorganismen, beispielsweise Hefe, feststellbar und beeinflußbar ist. Durch die erfindungsgemäß vorgeschlagene Regelung bzw. Steuerung der Temperatur der Maische im Behälter kann zuverlässig der Gärverlauf bzw. Gärvorgang beeinflußt bzw. geregelt oder gesteuert werden, wobei insbesondere eine Echtzeit- bzw. Online-Regelung der Temperatur vorgeschlagen wird. In einer einfachsten Form einer Verfahrensführung gelingt unter Berücksichtigung der Tatsache, daß nach Beginn des Gärvorgangs im wesentlichen lediglich CO₂ gebildet werden kann, nach einem ersten Ausbringen von Restluft aus einem Behälter die Feststellung der CO₂-Menge durch eine einfache Feststellung bzw. Messung, beispielsweise eine Durchflußmessung der aus dem Behälter austretenden Gasmenge, woraus unmittelbar die CO₂-Menge ermittelt werden kann, da gegebenenfalls zusätzlich enthaltene Bestandteile entweder vernachlässigbar oder ausreichend gut bekannt sind, so daß eine entsprechend zuverlässige Temperaturregelung möglich wird.

Zur weiteren Verbesserung der Verfahrensführung sowie zur präziseren Feststellung des Gärverlaufs bzw. der Gärintensität wird darüber hinaus vorgeschlagen, daß zusätzlich die Temperatur der Maische im Behälter gemessen wird. Eine derartige zusätzliche, für sich gesehen bekannte Ermittlung der Temperatur erlaubt eine weitere Präzisierung bei der Steuerung des Gärvorgangs bzw. des Gärverlaufs. Die zusätzliche Ermittlung der Temperatur ermöglicht ergänzend eine Einstellung eines optimalen Temperaturbereichs der zu regelnden Temperatur zur Durchführung bzw. Erzielung eines gewünschten Gärverlaufs.

Zusätzlich oder alternativ zur ergänzenden Feststellung der. Temperatur neben der Ermittlung insbesondere der CO₂-Menge und/oder des CO₂-Gehalts wird gemäß einer weiters bevorzugten Ausführungsform der Erfindung vorgeschlagen, daß der pH-Wert, L-Laktat-Wert, Zuckerwert, Leitwert und/oder die Dichte der Maische ermittelt wird und für die Regelung bzw. Steuerung des Gärvorgangs eingesetzt wird. Durch Ermittlung dieser Werte der Maische kann eine weitere Verbesserung bei der Regelung bzw. Steuerung des Gärverlaufs und/oder der Gärintensität erzielt werden.

Zur Erzielung des gewünschten Gärergebnisses wird im Zusammenhang mit der erfindungsgemäß vorgeschlagenen Feststellung der Gasmenge, insbesondere der CO₂-Menge und/oder des CO₂-Gehalts, bzw. der gegebenenfalls vorgesehenen Feststellung weiterer physikalischer Parameter vorgeschlagen, daß in Abhängigkeit von der Gasmenge, insbesondere der CO₂-Menge und/oder dem CO₂-Gehalt, und/ oder weiteren physikalischen Parametern zusätzlich der Druck des im Behälter befindlichen Gases geregelt bzw. gesteuert und/oder Hefe zu der Maische zugesetzt wird, wie dies einer weiters bevorzugten Ausführungsform des erfindungsgemäßen Verfahrens entspricht. In Abhängigkeit von den ermittelten CO₂-Werten sowie gegebenenfalls weiteren ermittelten Werten von zusätzlichen physikalischen Parametern läßt sich entsprechend genau der gewünschte Gärverlauf zur Erzielung eines gewünschten Ergebnisses durch die zusätzliche Regelung bzw. Steuerung des Drucks des im Behälter befindlichen Gases durchführen, wobei alternativ oder zusätzlich zur Unterstützung des Gärvorgangs der Maische Hefe zugesetzt werden kann.

Mit dem erfindungsgemäßen Verfahren ist hiebei nicht nur die Regelung bzw. Steuerung des Gärvorgangs bzw. Gärfortschritts möglich, sondern es läßt sich aus der Ermittlung der CO₂-Menge und/ oder des CO₂-Gehalts und der Ermittlung von gegebenenfalls weiteren bzw. zusätzlichen physikalischen Parametern auch ein für ein angestrebtes Ergebnis optimaler Zeitpunkt der Beendigung der Gärung festlegen, wobei in diesem Zusammenhang gemäß einer weiters bevorzugten Ausführungsform vorgeschlagen wird, daß in Abhängigkeit von der Gasmenge, insbesondere der CO₂-Menge bzw. dem CO₂-Gehalt, und/oder weiteren physikalischen Parametern der Gärvorgang unterbrochen bzw. abgebrochen wird.

Zur Lösung der oben angeführten Aufgaben ist darüber hinaus eine Vorrichtung der eingangs genannten Art im wesentlichen dadurch gekennzeichnet, daß ein Sensor zur Ermittlung der Gasmenge, insbesondere der CO₂-Menge bzw. des CO₂-Gehalts, des in dem Behälter vorhandenen bzw. sich bildenden Gases vorgesehen ist, welcher mit der Steuer- bzw. Regeleinrichtung gekoppelt ist. Derart wird eine konstruktiv einfache und kostengünstige Vorrichtung zur Verfügung gestellt, mit welcher eine zuverlässige Regelung bzw. Steuerung des Gärvorgangs bzw. Gärablaufs möglich ist.

Für eine konstruktiv besonders einfache und kostengünstige Ausbildung wird bevorzugt vorgeschlagen, daß der Sensor zur Ermittlung der Gasmenge, insbesondere der CO₂-Menge, von einem Durchflußsensor gebildet ist.

Zur Ermittlung weiterer physikalischer Parameter der Maische wird gemäß einer bevorzugten Ausführungsform vorgeschlagen, daß zusätzlich ein Temperatursensor zur Ermittlung der Temperatur der Maische vorgesehen ist. Darüber hinaus kann bevorzugt vorgesehen sein, daß zusätzlich wenigstens ein Sensor zur Erfassung des pH-Werts, L-Laktat-Werts, Zuckerwerts, Leitwerts und/oder der Dichte der Maische vorgesehen ist und mit der Steuer- bzw. Regeleinrichtung gekoppelt bzw. koppelbar ist.

Neben der Ermittlung genauerer Daten betreffend den Gärfortschritt bzw. Gärablauf durch Feststellung des CO₂-Gehalts des sich im Behälter über der Maische befindlichen Gases kann in Kenntnis des CO₂-Gehalts und gegebenenfalls weiterer physikalischer Parameter eine entsprechend gewünschte Beeinflussung des Gärvorgangs durchgeführt werden. In diesem Zusammenhang wird gemäß einer weiters bevorzugten Ausführungsform vorgeschlagen, daß die Steuer- bzw. Regeleinrichtung mit einer Einrichtung zum Regeln bzw.

Steuern der Temperatur der Maische und/oder des Drucks im Inneren des Behälters und/oder zur Zufuhr von Hefe in den Behälter gekoppelt ist. Für eine einfache Steuerung bzw. Einstellung der Temperatur wird hiebei vorgeschlagen, daß in an sich bekannter Weise wenigstens ein Wärmetauscher im Inneren des Behälters vorgesehen ist, wie dies einer weiters bevorzugten Ausführungsform der erfindungsgemäßen Vorrichtung entspricht.

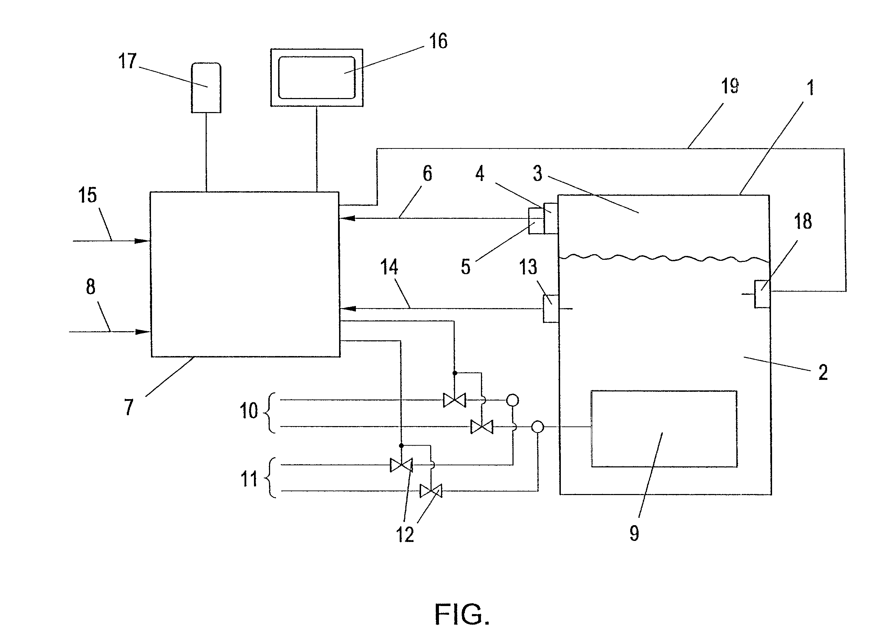

Die Erfindung wird nachfolgend anhand eines in der beiliegenden Zeichnung schematisch dargestellten Ausführungsbeispiels näher erläutert.

In der Figur ist mit 1 ein Behälter bzw. Gärtank bezeichnet, welcher mit einem Gärmaterial bzw. einer Maische 2 gefüllt ist. Über dem Gärmaterial bzw. der Maische 2 bildet sich bei Einleitung des Gärvorgangs ein insbesondere aus Luft und CO₂, welches bei der Gärung entsteht, bestehendes Gasgemisch im Raum 3, welches bei fortschreitender Gärung zunehmend durch ein nahezu vollständig aus CO₂ bestehendes Gasvolumen ersetzt wird, wobei dieses Gas über eine Austrittsöffnung 4 ausgebracht wird, an welcher beispielsweise ein Durchflußsensor 5 zur Bestimmung der Gasmenge, insbesondere der CO₂-Menge nach Beginn der Gärung, vorgesehen ist.

Der CO₂-Sensor 5 ist über eine Signalleitung 6 mit einer Steuer- bzw. Regeleinrichtung 7 gekoppelt, in welcher der vom Sensor 5 erhaltene CO₂-Wert bzw. die CO₂-Menge mit einem schematisch über eine Eingabe 8 eingegebenen Sollwert für den CO₂-Wert verglichen wird.

In Abhängigkeit von dem Vergleich zwischen dem durch den Sensor 5 ermittelten CO₂-Wert und dem über die Eingabe 8 eingegebenen Sollwert für den CO₂-Wert kann zur Steuerung bzw. Regelung des Gärverlaufs in weiterer Folge die Temperatur im Inneren des Behälters 1 und insbesondere der Maische 2 über einen schematisch mit 9 angedeuteten Wärmetauscher vorgenommen werden. Dieser Wärmetauscher kann entsprechend der zu erreichenden Temperatur über einen schematisch mit 10 bezeichneten Heißwasservorlauf und -rücklauf sowie mit 11 bezeichneten Kaltwasservorlauf und -rücklauf gespeist werden, wobei in den einzelnen Zuführungsleitungen eine Mehrzahl von Ventilen 12 angeordnet ist. Es läßt sich dadurch in einfacher Weise eine Echtzeit- bzw. Online-Temperaturregelung durchführen.

Bei einer abgewandelten Ausführung könnte der Sensor 5 unmittelbar den CO₂-Gehalt ermitteln lassen, wofür ein entsprechend aufwendigerer Sensor erforderlich wäre.

Zur weiteren Verbesserung der Steuerung und zur präziseren Einstellung des Gärverlaufs ist darüber hinaus ein Temperatursensor 13 angedeutet, welcher über eine Signalleitung 14 der Regel- bzw. Steuereinrichtung 7 ein Signal zur Verfügung stellt. Dieses Temperatursignal des Temperatursensors 13 wird wiederum mit einem über eine schematische Eingabe 15 angedeuteten Sollwert verglichen, so daß beispielsweise die Temperatursteuerung über den Wärmetauscher 9 nicht nur in Abhängigkeit von dem ermittelten CO₂-Wert, insbesondere der CO₂-Menge und/oder des CO₂-Gehalts, sondern auch unter Berücksichtigung des durch den Temperatursensors 13 ermittelten Temperaturwerts vorgenommen werden kann.

Mit der Steuer- bzw. Regeleinrichtung 7 können zusätzliche Peripheriegeräte verbunden sein, wobei beispielsweise ein Bildschirm 16 und eine Warneinrichtung mit 17 angedeutet ist.

Zusätzlich zu dem Temperatursensor 13 können gegebenenfalls weitere Sensoren insbesondere im Inneren des Behälters 1 vorgesehen sein, wobei wenigstens ein derartiger Sensor mit 18 angedeutet ist, welcher ebenfalls über eine schematisch mit 19 angedeutete Leitung mit der Steuer- bzw. Regeleinrichtung 7 verbunden ist. Mit einem derartigen zusätzlichen Sensor 18 kann beispielsweise der pH-Wert, L-Laktat-Wert, Zuckerwert, Leitwert und/oder die Dichte der Maische ermittelt werden und ebenso für die Regelung bzw. Steuerung des Gärvorgangs eingesetzt werden.

Zusätzlich zu der in der Figur dargestellten Steuerung bzw. Regelung der Temperatur der Maische 2 zur Steuerung des Gärvorgangs bzw. Gärverlaufs kann vorgesehen sein, den Druck des Gases im Raum 3 entsprechend zu regeln bzw. zu steuern, wobei zu diesem Zweck in der Austrittsöffnung 4 lediglich ein entsprechend steuerbares Ventil vorzusehen ist.

Alternativ oder zusätzlich kann zur weiteren Steuerung bzw. Beeinflussung des Gärverlaufs auch entsprechend eines in der Steuer- bzw. Regeleinrichtung 7 ermittelten Werts der Zusatz von Hefe in den Behälter 1 zur Beschleunigung des Gärvorgangs vorgenommen werden.

Neben einer Steuerung des Gärverlaufs bzw. Gärvorgangs kann selbstverständlich auch in Abhängigkeit von zumindest dem ermittelten CO₂-Wert der Gärvorgang abgebrochen bzw. unterbrochen werden, falls von der Steuer- bzw. Regeleinrichtung 7 entsprechende Signale ausgegeben werden.

Über gegebenenfalls vorzusehende, weitere Peripheriegeräte kann auch eine Fernsteuerung über oder eine Warnung an entsprechend entfernte Einrichtungen, beispielsweise über SMS oder Internet, erfolgen.

## Patentansprüche

1. Verfahren zum Behandeln von Maische, wobei von der in einen Behälter (1) gefüllten Maische (2) wenigstens ein physikalischer Parameter erfaßt wird und ein Gärverlauf der Maische (2) in Abhängigkeit von dem Parameter geregelt wird, wobei die Gasmenge, insbesondere die CO₂-Menge und/oder der CO₂-Gehalt, des sich im Behälter (1) über der Maische (2) befindlichen bzw. bildenden Gases während der Gärung gemessen wird, **dadurch gekennzeichnet, daß** in Abhängigkeit von der Gasmenge die Temperatur der Maische (2) im Behälter (1) geregelt bzw. gesteuert wird.

2. Verfahren nach Anspruch 1, **dadurch gekennzeichnet, daß** zusätzlich die Temperatur der Maische (2) im Behälter (1) gemessen wird.

3. Verfahren nach Anspruch 1 oder 2, **dadurch gekennzeichnet, daß** der pH-Wert, L-Laktat-Wert, Zuckerwert, Leitwert und/oder die Dichte der Maische (2) ermittelt wird und für. die Regelung bzw. Steuerung des Gärvorgangs eingesetzt wird.

4. Verfahren nach Anspruch 1, 2 oder 3, **dadurch gekennzeichnet, daß** in Abhängigkeit von der Gasmenge, insbesondere der CO₂-Menge bzw. dem CO₂-Gehalt, und/oder weiteren physikalischen Parametern zusätzlich der Druck des im Behälter (1) befindlichen Gases geregelt bzw. gesteuert und/oder Hefe zu der Maische (2) zugesetzt wird.

5. Verfahren nach einem der Ansprüche 1 bis 4, **dadurch gekennzeichnet, daß** in Abhängigkeit von der Gasmenge, insbesondere der CO₂-Menge bzw. dem CO₂-Gehalt, und/oder weiteren physikalischen Parametern der Gärvorgang unterbrochen bzw. abgebrochen wird.

6. Vorrichtung zum Behandeln von Maische in einem Behälter (1) mit wenigstens einem Sensor (5, 13, 18) zur Erfassung eines physikalischen Parameters der Maische (2) und einer Steuer- bzw. Regeleinrichtung (7) zur Beeinflussung des Gärverlaufes, **dadurch gekennzeichnet, daß** ein Sensor (5) zur Ermittlung der Gasmenge, insbesondere der CO₂-Menge bzw. des CO₂-Gehalts, des in dem Behälter (1) vorhandenen bzw. sich bildenden Gases vorgesehen ist, welcher mit der Steuer- bzw. Regeleinrichtung (7) gekoppelt ist.

7. Vorrichtung nach Anspruch 6, **dadurch gekennzeichnet, daß** der Sensor (5) zur Ermittlung der Gasmenge, insbesondere der CO₂-Menge, von einem Durchflußsensor gebildet ist.

8. Vorrichtung nach Anspruch 6 oder 7, **dadurch gekennzeichnet, daß** zusätzlich ein Temperatursensor (13) zur Ermittlung der Temperatur der Maische (2) vorgesehen ist.

9. Vorrichtung nach Anspruch 6, 7 oder 8, **dadurch gekennzeichnet, daß** zusätzlich wenigstens ein Sensor (18) zur Erfassung des pH-Werts, L-Laktat-Werts, Zuckerwerts, Leitwerts und/oder der Dichte der Maische (2) vorgesehen ist und mit der Steuer- bzw. Regeleinrichtung (7) gekoppelt bzw. koppelbar ist.

10. Vorrichtung nach einem der Ansprüche 6 bis 9, **dadurch gekennzeichnet, daß** die Steuer- bzw. Regeleinrichtung (7) mit einer Einrichtung (9) zum Regeln bzw. Steuern der Temperatur der Maische (2) und/oder des Drucks im Inneren des Behälters (1) und/oder zur Zufuhr von Hefe in den Behälter (1) gekoppelt ist.

11. Vorrichtung nach Anspruch 10, **dadurch gekennzeichnet, daß** in an sich bekannter Weise wenigstens ein Wärmetauscher (9) im Inneren des Behälters (1) vorgesehen ist.
